# EUROPÄISCHE PATENTSCHRIFT

(11) **EP 0 945 102 B1**
(45) Veröffentlichungstag und Bekanntmachung des Hinweises auf die Patenterteilung: **31.05.2006**
(21) Anmeldenummer: 99102671.7
(22) Anmeldetag: 12.02.1999
(51) Int. Cl.: A61B 5/145, G01N 33/86

(54) **Implantierbare diagnostische Vorrichtung**
Implantable measuring device
Dispositif de mesure implantable

(30) Priorität: 13.03.1998 DE 19811017
(43) Veröffentlichungstag der Anmeldung: 29.09.1999
(73) Patentinhaber: Dade Behring Marburg GmbH, 35001 Marburg (DE)
(72) Erfinder: Kraus, Michael Dr., 35041 Marburg (DE)

(56) Entgegenhaltungen:
- DE-A- 3 933 373
- US-A- 5 711 861
- US-A- 5 833 603

## Beschreibung

Die vorliegende Erfindung betrifft eine implantierbare diagnostische Vorrichtung zur Bestimmung von analytischen Parametern, die im wesentlichen besteht aus einer Mehrzahl von identischen und/oder unterschiedlichen Meßeinheiten zur Bestimmung eines analytischen Parameters in denen ein Signal erzeugt wird, das in einem bestimmten Verhältnis zu der zu bestimmenden Größe steht und das durch geeignete Maßnahmen auf einen außerhalb des Körpers befindlichen Empfänger übertragen wird.

lmplantierbare diagnostische Vorrichtungen wurden bereits beschrieben, z. B. als Bestandteile von implantierbaren Insulinpumpen. Im wesentlichen bestehen solche Vorrichtungen aus einer implantierbaren Meßkammer, in der ein Biosensor ein analytabhängiges Signal erzeugt, welches seinerseit zur Steuerung der Insulinpumpe dient. Diese Vorrichtungen müssen periodisch ausgetausch werden, wenn der Insulinvorrat erschöpft ist. Da dieser Austausch relativ häufig stattfindet, ist das Austauschinterval häufig kürzer als die Standzeit der Biosensoren.

Nachteilig bei den bisherigen Verfahren und wahrscheinlich auch ein Grund, warum die bisher Verfahren zur Glukosebestimmung in Zusammenhang mit Insulinpumpen nicht auf weitere Parameter übertragen worden sind, ist das Problem, das entstehen würden, wenn die Biosensoren über einen längeren Zeitraum mit Blut in Kontakt sind und dann ihre Funktion durch Ablagerungen, wie z. B. Fett- oder Proteinhaltige Ablagerungen (Gerinnsel) beeinträchtigt oder gar unmöglich gemacht wird.

Insbesondere Verfahren zur Bestimmung von Gerinnungsparametern, die naturgemäß oft mit einer Gerinnsel-Bildung einhergehen, werden als nicht aussichtsreich betrachtet.

US5 711 861 offenbart eine implantierbare Vorrichtung mit Enzymbeschichteten Elektroden. Des weiteren wird offenbart, daß die Vorrichtung über zwei Sensorflächen verfügt, von denen jede jeweils über mindestens ein Elektrodenpaar verfügt.

DE 39 33 373 offenbart eine implantierbare Vorrichtung deren Sensorelement aus einer in einer enzymhaltigen Matrix eingebetteten Platinelektrode besteht. Die Vorrichtung verfügt über eine Blutleitung, die unter der Messposition der Elektrode eine Öffnung aufweist, so dass ein Flüssigkeitskontakt zwischen Elektrodenmesskopf und Flüssigkeit hergestellt wird.

Die der vorliegenden Erfindung zugrunde liegende Aufgabe bestand darin, eine diagnostische Vorrichtung zu schaffen, die es ermöglicht, die Intervalle zwischen den Austauschen der Vorrichtungen wesentlich zu verlängern.

Dieses Ziel wird erfindungsgemäß durch eine Vorrichtung mit den Merkmalen des Anspruches 1 gelöst.

Überraschenderweise wurde gefunden, daß die erfindungsgemäße Vorrichtung, die mehrere Meßkammern enthält, vorteilhafterweise zur Bestimmung von Gerinnungsparametem eingesetzt werden kann.

Die erfindungsgemäße Vorrichtung besteht somit aus mehreren Meßkammern, die ihrerseits im wesentlichen aus drei Teilen besteht und zwar der eigentlichen Meßkammer, einer daraus hervorragenden Kanüle, die ihrerseits in ein Gefäß ragt, in dessen Flüssigkeitsvolumen der analytische Parameter nachgewiesen werden soll. Ferner gehört zu jeder Meßkammer - oder auch zu allen Meßkammern gemeinsam - eine Signalübertragungseinheit. Das gefäßseitige Ende der Kanüle kann vorteilhafterweise durch einen Filter abgeschlossen werden, der den Durchtritt der Analyten erlaubt aber Zellen zurückhält. Die Kanülen sind ferner mit einer Verschlußklappe versehen, die durch Einwirkung eines extrakorporalen Signals betätigt werden kann.

Die diagnostische Vorrichtung ist vorteilhafterweise zusätzlich mit einer Vorrichtung zur Speicherung der Meßsignale versehen, die dann bei Bedarf abgerufen werden können.

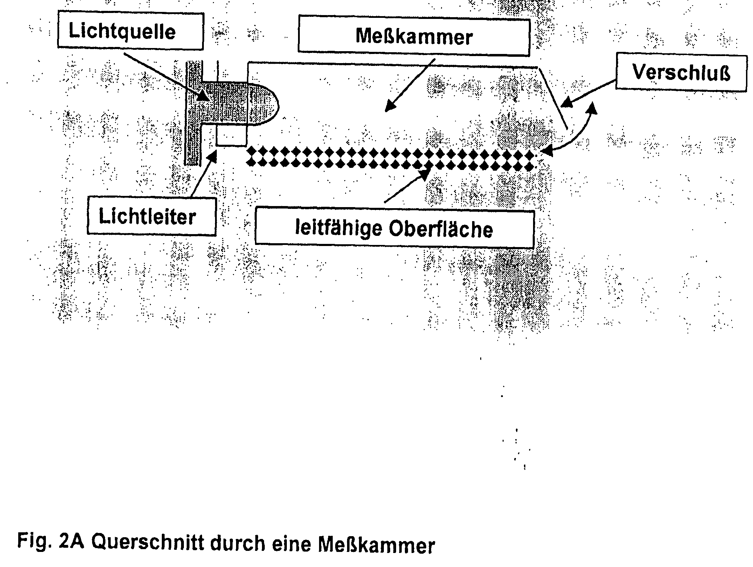

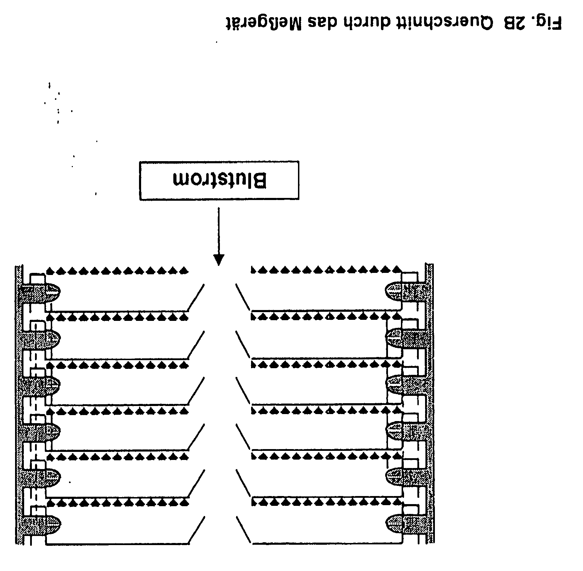

Eine bevorzugte Ausführungsform der Meßkammer ist in der Abbildung 2 dargestellt.

Vorteilhafterweise wird eine Mehrzahl dieser Meßkammern zu einer diagnostischen Vorrichtung zusammengefaßt, wobei die Anzahl der Einzelmeßkammern bevorzugterweise 20 bis 50 beträgt. Die Zahl als solche ist unkritisch, geht man von einer Bestimmung pro Woche aus, so erscheint eine Anzahl von 50 ± 10 besonders vorteilhaft, da in diesem Fall ein Austausch pro Jahr ausreichend ist.

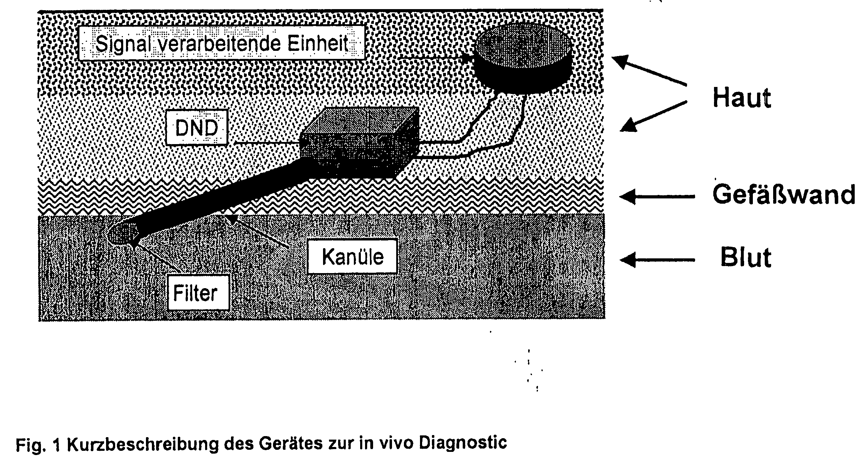

Das erfindungsgemäße Gerät kann sowohl für Durchflußmessungen als auch für Einzelbestimmungen ausgerüstet werden. Durch die Möglichkeit der kontaktlosen Signalübertragung kann es vorteilhafterweise sowohl bei stationären als auch ambulanten Patienten ohne eine zusätzliche Einschränkung der Beweglichkeit der Patienten eingesetzt werden. Die Arten der Signalübertragung sind dem Fachmann an sich bekannt. Der Aufbau einer Ausführungsform des Gerätes ist in Fig. 1 dargestellt. Der Aufbau einer speziellen Meßkammer ist in Fig. 2 A dargestellt. Die Herstellung ausreichend kleiner Meßkammern ist mit den dem Fachmann an sich bekannten Verfahren möglich.

Von besonderer Bedeutung ist der Verschluß der Meßkammern. Hier können vorteilhafterweise magnetisch betätigbare Verschlußklappen, die 2 feste Zustände einnehmen können (flip-flops) eingesetzt werden. In Fig. 2 B ist eine Anordnung von mehreren Meßkammern dargestellt. Die Lichtzufuhr zu den einzelnen Meßkammern kann dabei z.B. entweder über entsprechend kleine, für jede Meßkammer separat angeordnete Lichtquellen, z.B. Leuchtdioden, oder über entsprechende Lichtleiter erfolgen.

Vorteilhafterweise enthält jede Meßkammer das oder die für die Reaktion notwendige Reagenz(ien), Thromboplastin für eine PT (Thromboplastin-Zeit, "prothrombin time) Bestimmung. Mit den üblichen in der Gerinnung angewandten Bestimmungsverfahren ist die erfinderische Technologie schwierig zu verwirklichen. Vorteilhafterweise wird eine neue Technologie angewendet, die im folgenden beschrieben wird.

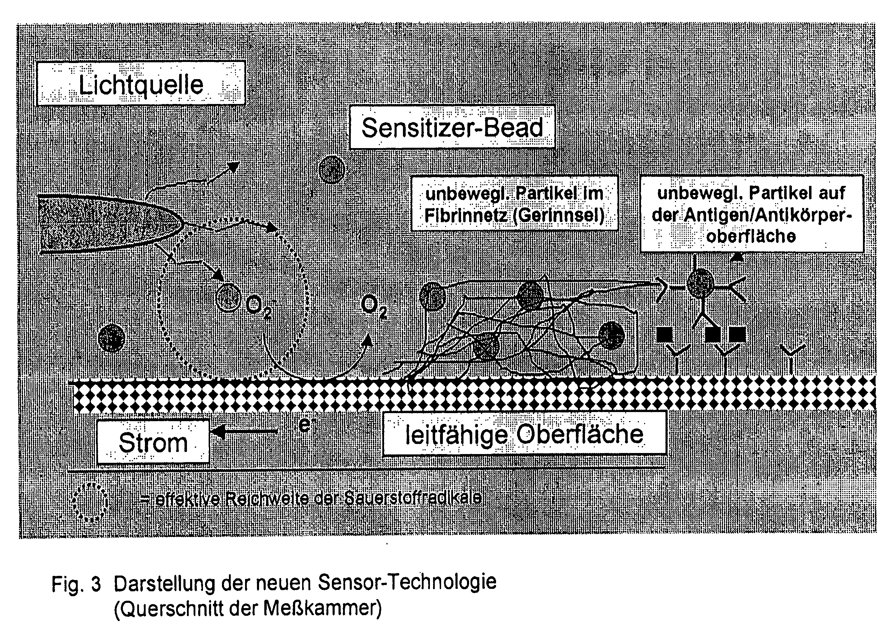

So können Singulett-Sauerstoff emittierende Partikel (SSEP), die z. B. in EP 0 515 194 beschrieben werden, verwendet werden. Diese SSEPs werden in der Meßkammer auf eine leitfähige Oberfläche zum Beispiel aus Kohlenstoff aufgebracht zusammen mit den analytspezifischen Reagenzien. In die Meßkammer ragt eine Lichtquelle, die ein Licht anstrahlt, das geeignet ist, die SSEPs anzuregen. Wenn eine Gerinnung erfolgt, wird die Beweglichkeit der SSEPs verändert, in der Regel eingeschränkt, dadurch häufen sich SSEPs an der leitfähigen Oberfläche an und es entsteht ein meßbarer Stromfluß (siehe auch Fig. 3).

Ein analoges Verfahren kann man für immunologische Bestimmungen anwenden, wobei ein spezifischer Bindungspartner auf der leitfähigen Oberfläche immobilisiert ist und durch den Analyten SSEPs, die ebenfalls mit einem, für den Analyten spezifischen Bindungspartner beschichtet sind, in der Nähe der leitfähigen Oberfläche gebunden werden, was wiederum zu einer meßbaren Veränderung des Stromflußes führt.

Kurze Beschreibung der Zeichnungen
- Abbildung 1: ist eine Kurzbeschreibung des Gerätes zur in vivo Diagnostic
- Abbildung 2A: zeigt einen Querschnitt durch eine Meßkammer
- Abbildung 2B: zeigt einen Querschnitt durch das Meßgerät
- Abbildung 3: ist eine Darstellung der neuen Sensor-Technologie (Querschnitt der Meßkammer)

Eine vorteilhafte Ausführungsform kann wie folgt ausgeführt sein:

Ein Gerät, bestehend aus den Elementen einer Meßeinheit, einer Signal verarbeitenden Einheit sowie einer Kanüle wird unter der Haut eingepflanzt, so daß die Kanüle in ein Gefäß ragt. Die Einheiten sind mittels Microtechnologie, wie sie beispielsweise bei der Herstellung integrierter Schaltkreise (Chip-Technologie) verwendet werden hergestellt und dadurch so klein, daß sie mit bekannten endoskopischen Techniken implantiert werden können. Zur Messung wird ein externer Receiver aufgelegt und der Strom zur Messung konduktiv übertragen. Gleichzeitig werden die Meßsignale bzw. Ergebnisse auf den Receiver übertragen und können dort abgefragt werden.

Die Meßeinheit besteht aus diskreten Meßkammern, die durch Ventile einzeln verschlossen werden können und nur einmalig verwendet werden können, sowie einer Pumpvorrichtung in Verbindung zur Kanüle, sowie ggfs. zu einem Vorratsbehälter mit physiologischer NaCl zum Spülen der Kanüle. Weiterhin enthält die Meßeinheit einen Lichtgenerator, etwa einen Mikrolaser, sowie Stromleitungen zur Ableitung und Verarbeitung des Meßsignals. Die Meßkammer enthält eine miniaturisierte Diode, die über einen Lichtleiter mit dem zentralen Lichtgenerator verbunden ist. In der Meßkammer befinden sich die zum Nachweis des Plasmaproteins bzw. zur Gerinnungsanalytik notwendige Reagenzien. Die Verfahren zur Herstellung der diskreten Bauelemente, wie z. B. Ventile oder Pumpen sind dem Fachmann an sich bekannt.

Der Nachweis der Reaktion erfolgt über eine neuartige Kombination von Sensitizer-Beads aus der LOCI-Technologie mit einer amperometrisch sensitiven Leiterplatine. Durch Anregung mit Licht werden Sauerstoff-Radikale erzeugt, die auf der Leiterplatine ein meßbares Signal erzeugen. Bei einer Gerinnselbildung entstehen diese aufgrund der Immobilisierung bevorzugt in Nähe der Leiterplatine. Die freie Lösung kann durch Rührer in Bewegung gehalten werden. Bei immunchemischen Reaktionen sind entsprechende Bindungspartner auf der Obertläche immobilisiert. Durch die Bindung der Sensitizer-Beads an den nachzuweisenden Analyten, der an den Bindungspartner der Oberfläche gebunden wird, werden die Sensitizer-Beads in die Nähe der Leiterplatine gebracht, so daß mit höherer Wahrscheinlichkeit eine Übertragung von Ladung (Sauerstoff-Radikale) erfolgt. Das Meßsignal ist somit der Analyt-Konzentration proportional.
Alternativ können auch gängige Reagenzien des LOCI-Systems, bestehend aus Sensitizer- und Akzeptor Beads verwendet werden. In der Meßkammer befindet sich dann ein Lichtempfänger anstelle der Leiterplatinen.

Die Signal verbeitende Einheit enthält einen Rechenprozessor sowie einen Transducer zur Übertragung der Ergebnisse, sowie weitere elektrische Einheiten zum Energieempfang.

Die Kanüle kann Filter enthalten, die den Ausschluß von zellulären Bestandteilen des Blutes gewährleisten, so daß Plasma zur Analyse verwendet wird. Durch rückwärtiges Spülen mit physiologischer Kochsalzlösung können nach Messung Zugangskanäle, Kanüle und Filter gesäubert werden.

## Patentansprüche

1. Implantierbare diagnostische Vorrichtung zur Bestimmung von analytischen Parametern, umfasend
a) eine Mehrzahl von identischen oder unterschiedlichen Meßkammern (Sensoren) zur Bestimmung eines analytischen Parameters,
wobei jede der Meßkammern Singutett-Sauerstoff emittierende Partikel enthält und in der Lage ist, ein Signal zu erzeugen, das in einem bestimmten Verhältnis zu der zu bestimmenden Größe/Parameter steht.
b) eine (Signalübertragungseinheit, die das Signal umsetzt und weiterleitet, und
c) eine Kanüle als Probenzuführung zur Meßeinheit

2. Vorrichtung nach Anspruch 1 **dadurch gekennzeichnet, daß** die Singulett-Sauerstoff emittierenden Partikel durch Licht anzuregen sind.

3. Vorrichtung Anspruch 1 oder 2 **dadurch gekennzeichnet, daß** die Singulett-Sauerstoff emittierenden Partikel mit einem analytspezifischen Bindungspartner beschichtet sind.

4. Vorrichtung nach Anspruch 1 oder 2 **dadurch gekennzeichnet, daß** in der Meßeinheit Reagenzien für die Bestimmung der Gerinnung enthalten sind.

5. Vorrichtung nach Anspruch 4 **dadurch gekennzeichnet, daß** in der Meßeinheit Reagenzien für die Bestimmung der Thromboplastin-zeit enthalten sind.

6. Vorrichtung nach Anspruch 1, **dadurch gekennzeichnet, daß** sie ein Außenmaß von 1500 µl, besonders bevorzugt von 300 µl nicht überschreitet.

7. Vorrichtung nach einem der Ansprüche 1 bis 6, **dadurch gekennzeichnet, daß** die Vorrichtung aus einer Mehrzahl von Meßkammern besteht, die zur Bestimmung desselben Parameters geeignet sind.

8. Vorrichtung nach einem der Ansprüche 1 bis 6 , **dadurch gekennzeichnet, daß** die Vorrichtung aus einer Mehrzahl von Meßkammern besteht, die zur Bestimmung unterschiedlicher Parameter geeignet sind.

9. Vorrichtung nach einem der Ansprüche 1 bis 8, **dadurch gekennzeichnet, daß** die zur Signalerzeugung und/oder Übertragung notwendige Energie durch eine mitimplantierte Batterie erzeugt wird.

10. Vorrichtung nach einem der Ansprüche 1 bis 8, **dadurch gekennzeichnet, daß** die zur Signalerzeugung und/oder Übertragung notwendige Energie durch einen externen Transducer zugeführt wird.

## Claims

1. Implantable diagnostic apparatus for determining analytic parameters, comprising:
a) a plurality of identical or different measurement chambers (sensors) for determining an analytic parameter,
each of the measurement chambers containing singlet oxygen emitting particles and being able to generate a signal which is specifically related to the variable/parameter to be determined,
b) a signal transmission unit, which converts the signal and forwards it, and
c) a cannula as sample feeder to the measurement unit.

2. Apparatus according to Claim 1, **characterized in that** the singlet oxygen emitting particles are to be excited by light.

3. Apparatus according to Claim 1 or 2, **characterized in that** the singlet oxygen emitting particles are coated with an analyte-specific binding partner.

4. Apparatus according to Claim 1 or 2, **characterized in that** the measurement unit contains reagents for determining the coagulation.

5. Apparatus according to Claim 4, **characterized in that** the measurement unit contains reagents for determining the prothrombin time.

6. Apparatus according to Claim 1, **characterized in that** it does not exceed an external dimension of 1500 µl, particularly preferably of 300 µl.

7. Apparatus according to one of Claims 1 to 6, **characterized in that** the apparatus comprises a plurality of measurement chambers which are suitable for determining the same parameter.

8. Apparatus according to one of Claims 1 to 6, **characterized in that** the apparatus comprises a plurality of measurement chambers which are suitable for determining different parameters.

9. Apparatus according to one of Claims 1 to 8, **characterized in that** the energy necessary for signal generation and/or transmission is generated by a concomitantly implanted battery.

10. Apparatus according to one of Claims 1 to 8, **characterized in that** the energy necessary for signal generation and/or transmission is fed in by an external transducer.

## Revendications

1. Dispositif diagnostique implantable destiné à l'évaluation de paramètres analytiques, comprenant :
a) une pluralité de chambres de mesure identiques ou différentes (capteurs) pour l'évaluation d'un paramètre analytique,
dans laquelle chaque chambre de mesure contient une particule émettrice d'oxygène singulet et peut produire un signal qui entretient un rapport déterminé avec la grandeur/le paramètre à évaluer,
b) une unité de transmission du signal qui convertit le signal et le transmet, et
c) une canule pour introduction de l'échantillon dans l'unité de mesure.

2. Dispositif selon la revendication 1, **caractérisé en ce que** la particule émettrice d'oxygène singulet doit être excitée par la lumière.

3. Dispositif selon la revendication 1 ou la revendication 2, **caractérisé en ce que** la particule émettrice d'oxygène singulet est revêtue d'un partenaire de liaison spécifique de l'analyte.

4. Dispositif selon la revendication 1 ou la revendication 2, **caractérisé en ce que** des réactifs de détermination de la coagulation sont contenus dans l'unité de mesure.

5. Dispositif selon la revendication 4, **caractérisé en ce que** des réactifs de détermination du temps de thromboplastine sont contenus dans l'unité de mesure.

6. Dispositif selon la revendication 1, **caractérisé en ce que** sa contenance ne dépasse pas 1 500 µl, de manière particulièrement préférée ne dépasse pas 300 µl.

7. Dispositif selon l'une quelconque des revendications 1 à 6, **caractérisé en ce que** le dispositif est constitué d'une pluralité de chambres de mesure conçues pour l'évaluation du même paramètre.

8. Dispositif selon l'une quelconque des revendications 1 à 6, **caractérisé en ce que** le dispositif est constitué d'une pluralité de chambres de mesure conçues pour l'évaluation de paramètres différents.

9. Dispositif selon l'une quelconque des revendications 1 à 8, **caractérisé en ce que** l'énergie nécessaire à la production du signal et/ou à sa transmission est produite par une pile implantée avec le dispositif.

10. Dispositif selon l'une quelconque des revendications 1 à 8, **caractérisé en ce que** l'énergie nécessaire à la production du signal et/ou à sa transmission est apportée par un transducteur externe.
